Europäisches Patentamt

European Patent Office

Office européen des brevets

(19)

(11) EP 0 567 564 B1

(12)
# EUROPEAN PATENT SPECIFICATION

(45) Date of publication and mention
of the grant of the patent:
**16.10.1996 Bulletin 1996/42**

(21) Application number: **92904426.1**

(22) Date of filing: **16.01.1992**

(51) Int Cl.[6]: **G01N 33/48**, G01N 27/28,
G01N 27/416, G01N 30/64,
G01N 33/68

(86) International application number:
**PCT/US92/00375**

(87) International publication number:
**WO 92/13273 (06.08.1992 Gazette 1992/21)**

(54) **Method for diagnosing disorders from biochemical profiles**

Verfahren zur Diagnose von Störungen der Körperfunktionen unter Verwendung von biochemischen Pryfilen

Méthode de diagnostic de dysfonctionnements organiques à partir de profils biochimiques

(84) Designated Contracting States:
**DE FR GB IT**

(30) Priority: **18.01.1991 US 643541**
**01.02.1991 US 649676**

(43) Date of publication of application:
**03.11.1993 Bulletin 1993/44**

(73) Proprietor: **ESA, Inc.**
**Bedford Massachusetts 01730 (US)**

(72) Inventor: **MATSON, Wayne, R.**
**Ayer, MA 01433 (US)**

(74) Representative: **Poulin, Gérard et al**
**Société de Protection des Inventions**
**25, rue de Ponthieu**
**75008 Paris (FR)**

(56) References cited:
**EP-A- 0 371 517**          **WO-A-86/03006**

- **J. TAYLOR: "Dorland's Illustrated Medical Dictionary", 27th edition, 1988, W. B. Saunders Co., Philadelphia, PA, US, see page 461: "Diagnosis"**

**Description**

This invention relates to analytical methods for diagnosing disorders. The invention has particular utility for diagnosing or categorizing disorders in living animals from analysis profiles of biologically active materials such as neurotransmitters and other neurochemical substances in brain tissue, cerebrospinal fluid, plasma, serum, saliva, nasal mucosa, urine and the like, such as catecholamines, their precursors, cofactors and their metabolites. The invention is uniquely capable of differentiating a large number of compounds of biological, diagnostic and/or pharmaceutical significance and of using such differential for diagnosing disorders and will be described in connection with such utility although other uses are contemplated.

There is an extensive body of literature relating abnormalities in neurotransmitters, precursors, and metabolites to degenerative, neuropsychiatric and behavioral disorders, hypertension and certain carcinomas. See, for example, Schildkraut et al in The Brain, Biochemistry and Behavior, Proceedings of the Sixth, Arnold O. Beckman Conference in Clinical Chemistry, pages 47-68. Although the potential role of these compounds in a number of significant disorders has been established, their routine analysis has not yet achieved widespread clinical use. Two problems in the clinical utility of neurotransmitter measurements are related to the economic and technical limitations of current technology. First, there is felt to be a high degree of interlaboratory and intersample uncertainty in quantitative values. Second, it has been difficult to measure enough of the known metabolically related compounds of a particular neurotransmitter to fully describe its biochemical significance in an individual sample, or to detect, identify and measure unusual neurotransmitters - an important aspect of basic research in various disease states that is presently very expensive and specialized.

While a number of interlaboratory technique intercomparisons for a variety of neurotransmitters have been carried out, there has been no comprehensive study within and among different techniques and laboratories for neurotransmitters in typical samples of interest. In the absence of such studies, given the complexity of the analytical problem and the historically wide variation whenever an analyte has been subjected to rigorous interlaboratory testing, the current values for normal and abnormal neurotransmitter levels must be take with unspecified and probably wide limits of confidence.

Although the analysis of single neurotransmitters or metabolites from a complex biochemical pathway has been shown to correlate with a number of disorders utilizing statistical analysis over a large number of samples, the analytical level of a single neurotransmitter in an individual sample, with a few exceptions, has had relatively low clinical diagnostic utility. Essentially the state of the field of biochemical correlates of disorders is that while between large populations of normal and abnormal individuals a correlation generally can be determined for a particular biochemical, the scatter that results from both analytical and biochemical phenomena typically does not permit the level of a particular biochemical to be utilized diagnostically for a particular single individual. Nor may a single biochemical value be utilized for the rational prescription or development of a pharmaceutical for that individual. This is not particularly surprising in that both the levels and effects of a particular neurotransmitter are modified by a number of other neurotransmitters, in the same, or parallel metabolic pathways. If, for instance, 5-HT (serotonin) is to be used as a diagnostic tool for depression, suicidal tendencies, or schizophrenia, it would be necessary and perhaps provide a route to definitive diagnosis and pharmaceutical specification or development, to simultaneously determine the approximately 40 other compounds that derive from tryptophan and significantly effect the indolaminergic neuronal system's activity.

In recent years, LCEC (Liquid Chromatography with Electrochemical Detection) has become a common tool for the determination of catecholamines biogenic amines and their metabolites in biological fluids. Because of sensitivity limitations (typically 20-50 pg) and the complexity of biological samples, both separation and concentration steps typically have been necessary. Heretofore, plasma catecholamine analysis typically required three steps. First, the sample is collected and the catecholamines separated and concentrated, for example, using the alumina extraction procedure of Anton and Sayre (See A.H. Anton and D.F. Sayre, J. Pharmacol, Exp. Ther., 138 (1962), p. 360-375). The analytes, norepinephrine, epinephrine and dopamine, along with the internal standard DHBH (dihydroxybenzylamine), then are separated chromatographically, and finally detected electrochemically. Typical sample size requirements are 1.0 ml plasma or serum. In routine clinical use, there have been numerous problems with conventional techniques (alumina absorption, ion exchange and extraction), due to a large number of poorly understood variables, in the overall analysis system of sample acquisition, storage, preparation and sensor response. These problems have quite likely confused the relationships that may exist between levels and distribution of the catecholamines and various physiological and behavioral phenomena and disease states.

In the analysis of complex biological materials such as blood, serum and cerebrospinal fluids which may contain numerous different constituents, the important (e.g. abnormal) metabolites such as neurotransmitters to be identified may be present in only parts per trillion. While a chromatographic column can achieve macro separation of the various constituents, it may not provide adequate spatial (in time) separation of the extremely small portion of metabolites of interest from the much larger percentage of the many other compounds coeluted from the column at the same time as the metabolites of interest. Many of these interfering coeluted materials are electrochemically active but electrochemically irreversible, while many metabolites such as neurotransmitters are both electrochemically active and electrochemically reversible. It has been found that the analytical problems of reliable measurements of neurochemicals and related compounds are complicated by the fact that interferences with conventional or prior technologies are disorder related. This was discussed in a prior publication, (Matson et al, Clinical Chemistry, Vol. 30, No. 9, 1984) (see U.S. Patent 4,511,659) for dopamine, dopac and seratonin measurements in directly analyzed brain extract and cerebrospinal fluid for normal, schizophrenics and Alzheimers. Recent work has indicated that even for the widely used and accepted technique of alumina extraction for plasma catecholamines that inferences may be disorder specific. Higher values for Norepinephrine (NE) and Epinephrine (EP) were observed following alumina extraction and analysis of a single energy conventional electrochemical detector than for a three cell redox detector on neonatal stress blood samples. Analysis of the neonate extracts on the sixteen channel chemical imaging system revealed several unexpected compounds that are potential interferences including dihydroxyphenylacetic acid (DOPAC), 3 hydroxykynurenamine (3-OHKYA) and 3-hydroxy-anthranilic acid (3-OHAN). These compounds have not been detected in normal adult plasma alumina extracts.

In the aforesaid U.S. Patent No. 4,511,659, there is provided an electrochemical detection system comprising a plurality of coulometrically efficient electrochemical cells, in series, for sequentially oxidizing and reducing selected substances in a sample solution under controlled conditions prior to measurement on a downstream testing electrode or electrodes. More specifically, in accordance with the invention provided in the aforesaid U.S. Patent No. 4,511,659, a sample solution (e.g. a body fluid) is passed through a suitable chromatographic column and the eluant is streamed in contact with a series of electrochemically isolated, in-line coulometric electrodes operated under conditions so as to establish a series of "gates" for the sequential oxidation and reduction of substances in the sample solution whereby to screen (remove) selected interfering and electrochemically irreversible substances contained in the sample solution, while passing selected electrochemically reversible products for detection and measurement on a downstream electrode. The gate electrode series is follows in-line by one or more, preferably an array of six or more coulometric measuring electrodes, each formed of porous electrode base material such as fritted graphite, fritted carbon or other conductive fritted material, for detecting and measuring the electrochemically reversible compounds of interest (e.g. neurotransmitters).

As reported in the aforesaid U.S. Patent No. 4,511,659, there are several beneficial effects of this approach to electrochemical analysis. Long-term drift in response is effectively eliminated by acquiring essentially 100% of the signal. The capability of analyzing essentially 100% of a material allows the assay of compounds of unknown purity by relating them to the basic principles of electrochemical reaction embodied in Faraday's Law. Poisoning of the electrode, a dominant problem with electrochemical sensors, is effectively eliminated by the use of a much larger relative surface area for reaction. And, finally, and most important to the eventual development of array and gate cells, a coulometric electrode by virtue of its essentially 100% efficiency allows sequential oxidation and/or reduction of compounds at successive-in-line detectors. The improved sensitivity of the detection system as discussed in the aforesaid U.S. Patent No. 4,511,659, particularly where two or more active testing electrodes follow the screening electrodes has given the ability to do direct injections of serum filtrates and has also allowed the generation of reproducible patterns of compounds with catecholamine like electrochemical behavior of a large number of resolvable components. This provides the possibility of performing pattern recognition for the diagnosis or perhaps even predictive diagnosis, of various disorders or disease states.

U.S. Patent 4,863,873, describes a system for resolving and detecting hundreds of compounds in a single sample at femtogram levels whereby to provide a small molecule inventory or metabolic pathway pattern of an individual. As taught in the aforesaid U.S. Patent 4,863,873, the small molecule inventory may be considered to reflect the underlying activity and distribution of the enzymatic pathways of an individual and hence reflect an operational measure of the genome determining those enzymes. The small molecule inventory of an individual may thus be used to determine the health state of the individual and/or to diagnose disease states. Correlation of the patterns from a plurality of individuals provides an understanding of the mechanisms of disorders or disease states or conditions and, in turn, provides a rational route to pharmacological development leading to treatment, cure or suppression of such disorders, disease states or conditions.

The present invention is an improvement in the invention described in the aforesaid U.S. Patent 4,863,873. More particularly, in the practice of the invention as described in the U.S. Patent 4,863,873, it has been observed that the biochemical analysis profiles of "normal" or healthy individuals may vary quite widely, while the biochemical profile analysis data of individuals having disorders is far less chaotic. More particularly, it has been observed that the frequency distribution of certain biochemical compounds or ratios of compounds in individuals suffering from a disorder are far less chaotic than "normal" or healthy individuals. This leads to a general protocol for diagnosing, categorizing or differentiating individuals based on comparisons of biochemical analytical data of small molecule inventory against data bases of known or previously diagnosed cases. By way of example the process of the present invention may advantageously be employed in the differentiation of neurological degenerative dementing or affective disorders such as Alzheimer's disease, Huntington's disease, Parkinson's disease, schizophrenia, Progressive Supernuclear Palsy, amyotrophic lateral sclerosis and senile dementias from each other and neurologically normal controls. Moreover, by suitable selection of variables, the process of the present invention also is applicable to classification of tumors, carcinomas, cardiovascular abnormalities and other disorders. Similarly, the process of the present invention advantageously may be utilized to select therapy based on categories of known successful vs. unsuccessful outcomes.

While not wishing to be bound by theory, the two fundamental hypotheses underlying the process of the present invention are:

1. The underlying genetic makeup or predisposition of an individual will reflect through the proteins, enzymes, and other factors it determines in patterns of small molecules. Individual components within these patterns will be affected by environmental effects such as diet, stress or chemical inset; however, the overall pattern of relationships will reflect the underlying operation of the genome or the interference of a particular disorder. Among the small molecules are the transmitters, cofactors and metabolites that regulate neuronal and endocrine functions and the interactions of somatic and central nervous system processes. Thus, the compounds such as purines, tyrosine and tryptophan derived neurotransmitters, peptides, pterin and vitamin cofactors are highly relevant to the effect or etiology of neurological disorders, cardiovascular disfunction and certain tumors or carcinomas.

2. The relationships of these biochemical patterns from a disorder are less chaotic or more regular than those from healthy controls. All of the biochemical systems of small molecules are interconnected and interrelated in a complex web of feedback and response. These interactions are highly nonlinear and thus, depending on subtle differences in initial conditions, the response of individual components in a biochemical pattern will be highly variable. The overall system will thus behave in a mathematically chaotic fashion. In a disorder, elements within the biochemical pattern are over or underregulated, thus reducing the degrees of freedom or overall variability. Consequently, the presence of a disorder implies more regulated or less chaotic variability of compounds or relationships among compounds in patterns from disordered individuals.

These two fundamental hypotheses provide an approach to diagnostic categorization of disorders using frequency distributions of compounds and relationships from large data bases.

<u>Description of the Drawings</u>

For a fuller understanding of the nature and objects of the present invention, reference should be made to the following detailed description taken in combination with the accompanying drawings wherein:

Figs. 1a-1d are frequency distribution graphs of variables of Alzheimer's Disease and controls;
Fig. 2 is a geographical representation of scoring algorithms;
Fig. 3 is a plot showing initial scoring of Alzheimer's Disease v. control;
Fig. 4 is a plot similar to Fig. 3, with five Alzheimer's Disease cases which scored as controls removed from the Alzheimer's Disease scoring Distribution;
Fig. 5 is a plot showing analog distribution measurements in accordance with the present invention;
Fig. 6A is a plot of measurements of nasal mucosa swab samples at low gain;
Fig. 6B is a plot of nasal mucosa swab samples at high gain; and
Fig. 7 is a plot of $\beta$-amyloid in saline suspension of mucosa swab.

<u>Detailed Description of the Invention</u>

<u>EXAMPLE I</u>

<u>Methodology for Sample Analysis and Data Base Creation</u>

280 CSF samples from the categories Alzheimer's Disease - AD (61 samples), Parkinson's Disease - PD (60 samples), schizophrenia - SC (60 samples), Huntington's Disease - HD (20 samples), Supernuclear Palsy - PSP (13 samples) and neurologically normal controls - C (68 samples), were electrochemically analyzed in accordance with the teachings of the aforesaid U.S. Patent 4,863,873. Samples from normal and diseased individuals were prepared and flowed through a chromatographic column, and detected in a 16 sensor electrochemical cell using an NCA Chemical Analyzer, Model No. CEAS available from ESA, Inc., Bedford, Massachusetts. Sensor potentials ranged from $T_1$ -600mv to $T_{16}$ +900mv in 100mv increments. All samples were from 7th or 8th mL aliquots of rostral caudal gradients. Pools were created for each category utilizing small subaliquots of the samples, and pools of all samples were created for analytical quality control and evaluation of unknowns. Samples were run under a variant of a standard reverse phase gradient procedure in the repetitive sequence Control Standard, Pool, 7 Samples, Control Standard, Pool, ...

as set forth in Table 1:

TABLE 1. CHARACTERISTICS OF METHOD 1.

Real time set up review; Times and events in the methods chromatographic functions.
Review of live method: Potentials and currents of channels 1-16 range functions temperature and limits.

## Table 1

```
                        REAL TIME SETUP REVIEW

            TIME        DEVICE        FUNCTION        VALUE       TOTAL
     1       0.00        FLOW           %B              5          1.20
     2       0.20      CLEAN CELL       ON             960
     3       0.50      CLEAN CELL      OFF
     4       7.00      AUTO ZERO        ON
     5       7.20.      FLOW           %B               5          1.20
     6       7.58    AUTO SAMPLER     INJECT
     7       8.00       FILE          START
     8      66.00       FLOW           %B              94          1.06
     9      66.00       FLOW           %B               5          1.20
    10      70.00       FILE          STOP
    11      70.00       FLOW           %B               5          1.20
    12      70.00    AUTO SAMPLER      STEP
    13      70.00       END                                        2
    14      70.00      METHOD        HI47
    15
    16
    17
    18
    19                                                                      EXIT
    20
```

```
                        Review of Live Method

       Full Scale Current                 P stats                      Autorange
                                                                          On
    10uA 100uA   10uA   10uA    -40mV    25mV    90mV    155mV
     1uA   1uA   10uA    1uA    220mV   285mV   350mV    415mV          Floor
     1uA   1uA    1uA    1uA    480mV   545mV   610mV    675mV          100nA
     1uA   1uA    1uA    1uA    740mV   805mV   870mV    910mV

    Cell Box Temp : 35.0 C

                                               UPPER LIMIT   LOWER LIMIT

                                     PUMP A:       350            0
                                     PUMP B:       350            0

                                     VALVE: Pos 1
                          8
```

```
    Exit                                     Display Time Line
```

Mobile Phase:

    A:   0.05 M LiPO$_4$, pH 2.86, 3 mg/L Dodecyl Sulfonic Acid
    B:   30% MeOH, 0.05 M  LiPO$_4$, pH 2.86, 3 mg/L Dodecyl Sulfonic Acid

Column:

    Dual 5u ODS, 4.6 mm x 15 cm ESA NBS

Samples were analyzed for 38 known components (listed in Table 2) and for 18 well-defined unknown peaks that were isolated in all pools shown in Table 3. (Asterisks denote components used in evaluation of regression and cluster analysis statistical procedures for categorization of groups.)

Table 2

Oracle compatible record showing retention times, digitized characteristic responses across channels, and set of control standard. Abbreviations are described in Table 6.

Table 2

Table: NOV8STD
Study: CSFRUN01
# Identified Compounds in Standard : 38
# Missing Compounds in Standard : 0

| # | File / RT | Name / Conc | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|---|---|
| 1 | std0001 | ASC | 1.717 | 1.700 | 1.725 | 1.725 | 1.750 | 1.733 | | | | | | | | | | |
| | 1.725 | 10000.00 | 3872279 | 9625125 | 1.27e+07 | 1.60e+07 | 1.50e+07 | 1.14e+07 | | | | | | | | | | |
| *2 | std0001 | CTS | | | | | | | 2.267 | 2.267 | 2.258 | 2.267 | 2.275 | 2.283 | 2.292 | | | |
| | 2.283 | 1000.00 | | | | | | | 129467 | 251043 | 528682 | 1298734 | 1601456 | 2250767 | 1062584 | | | |
| *3 | std0001 | URIC | | | 2.333 | 2.342 | 2.350 | 2.350 | | | | | | | | | | |
| | 2.350 | 8000.00 | | | | 5672 | 1324197 | 3193191 | 1019120 | | | | | | | | | |
| *4 | std0001 | XAN | | | | | | | | | 3.042 | 3.033 | 3.058 | 3.067 | 3.067 | 3.108 | | |
| | 3.058 | 1000.00 | | | | | | | | | 47171 | 1393667 | 3304889 | 2039171 | 358667 | 110609 | | |
| *5 | std0001 | HX | | | | | | | | | | | | | | 3.108 | 3.117 | 3.125 |
| | 3.125 | 100.00 | | | | | | | | | | | | | | 110609 | 154070 | 161138 |
| *6 | std0001 | VMA | | | | 4.083 | 4.067 | 4.075 | 4.075 | | | | | | | | | |
| | 4.075 | 10.00 | | | | 387 | 4959 | 16674 | 13957 | | | | | | | | | |
| *7 | std0001 | GSH | | | | | | | 4.875 | 4.875 | 4.875 | 4.875 | 4.892 | 4.900 | 4.900 | | | |
| | 4.900 | 500.00 | | | | | | | 13957 | 28075 | 51532 | 117237 | 133772 | 197987 | 85618 | | | |
| 8 | std0001 | HE | 5.625 | 5.642 | 5.642 | 5.650 | 5.650 | | | | | | | | | | | |
| | 5.642 | 10.00 | 334 | 18928 | 17463 | 1507 | 117 | | | | | | | | | | | |
| *9 | std0001 | HHPG | | | | | 6.075 | 6.075 | 6.075 | 6.075 | 6.083 | 6.092 | | | | | | |
| | 6.075 | 10.00 | | | | | 749 | 13983 | 5545 | 1828 | 1718 | 1044 | | | | | | |
| 10 | std0001 | HGA | 6.392 | 6.408 | | | | | | | | | | | | | | |
| | 6.392 | 10.00 | 16607 | 4976 | | | | | | | | | | | | | | |
| *11 | std0001 | G | | | | | | | | | 7.942 | 7.942 | 7.950 | 7.958 | 7.958 | | | |
| | 7.942 | 100.00 | | | | | | | | | 2395 | 122100 | 53287 | 4420 | 975 | | | |
| *12 | std0001 | GR | | | | | | | | | | 9.142 | 9.133 | 9.125 | 9.142 | 9.150 | 9.175 | 9.192 |
| | 9.150 | 500.00 | | | | | | | | | | 859 | 26558 | 194121 | 430593 | 1054244 | 189936 | 111915 |
| *13 | std0001 | LD | 9.325 | 9.283 | 9.283 | 9.292 | 9.275 | | | | | | | | | | | |
| | 9.283 | 10.00 | 165 | 18336 | 14158 | 1566 | 562 | | | | | | | | | | | |
| *14 | std0001 | HET | | | | | | | | | 9.642 | 9.642 | 9.650 | 9.650 | 9.650 | 9.667 | 9.675 | |
| | 9.650 | 500.00 | | | | | | | | | 986 | 6461 | 26013 | 33548 | 107832 | 86595 | 31459 | |
| 15 | std0001 | AN | | | | 10.008 | 10.008 | 10.017 | 10.000 | | | | | | | | | |
| | 10.008 | 100.00 | | | | 33745 | 118573 | 17512 | 2128 | | | | | | | | | |
| 16 | std0001 | EPI | 10.167 | 10.142 | 10.150 | | | | | | | | | | | | | |
| | 10.142 | 10.00 | 367 | 14152 | 9783 | | | | | | | | | | | | | |
| *17 | std0001 | A | | | | | | | | | | | | | | | 10.250 | 10.217 |
| | 10.217 | 100.00 | | | | | | | | | | | | | | | 26046 | 36349 |
| *18 | std0001 | DOPAC | 11.825 | 11.833 | 11.833 | 11.842 | 11.850 | 11.833 | | | | | | | | | | |
| | 11.833 | 10.00 | 247 | 7794 | 9749 | 1412 | 445 | 251 | | | | | | | | | | |
| 19 | std0001 | 3OMAN | | 13.225 | 13.200 | 13.217 | 13.242 | 13.250 | | | | | | | | | | |
| | 13.200 | 10.00 | | 1194 | 29905 | 8215 | 2107 | 1401 | | | | | | | | | | |
| *20 | std0001 | 3OMET | | 13.225 | 13.200 | 13.217 | 13.242 | 13.250 | | | | | | | | | | |
| | 13.200 | 10.00 | | 1194 | 29905 | 8215 | 2107 | 1401 | | | | | | | | | | |
| *21 | std0001 | 4HPLA | | | | | | | | | 13.442 | 13.450 | 13.467 | 13.458 | | | | |
| | 13.450 | 100.00 | | | | | | | | | 13364 | 125434 | 43118 | 2233 | | | | |
| *22 | std0001 | IAA | | | | 13.683 | 13.692 | 13.683 | | | | | | | | | | |
| | 13.692 | 10.00 | | | | 1593 | 14780 | 3917 | | | | | | | | | | |
| 23 | std0001 | 4HBAC | | | | | | | | | | | | | 13.983 | 13.992 | 13.992 | 14.008 |
| | 13.992 | 200.00 | | | | | | | | | | | | | 75738 | 207495 | 311864 | 170423 |
| *24 | std0001 | TYR | | | | | | | 15.250 | 15.242 | 15.225 | 15.233 | 15.250 | 15.250 | 15.292 | | | |
| | 15.233 | 1000.00 | | | | | | | 232 | 3866 | 102584 | 1181673 | 776764 | 117510 | 13964 | | | |
| *25 | std0001 | 5HTP | | 15.583 | 15.592 | 15.617 | | | | | | | | | | | | |
| | 15.592 | 10.00 | | 6994 | 16297 | 463 | | | | | | | | | | | | |
| 26 | std0001 | DA | 17.342 | 17.342 | 17.358 | 17.367 | 17.375 | | | | | | | | | | | |
| | 17.342 | 10.00 | 17722 | 6301 | 305 | 144 | 83 | | | | | | | | | | | |

Table 2 (Cont'd.)

| | File / RT | Name / Conc | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|---|---|
| *27 | std0001 | 5HIAA | 17.850 | 17.892 | 17.892 | 17.908 | 17.925 | | | | | | | | | | |
| | 17.892 | 50.00 | 233 | 9330 | 119759 | 14604 | 549 | | | | | | | | | | |
| *28 | std0001 | 4HPAC | | | | | | | 18.083 | 18.117 | 18.092 | 18.100 | 18.125 | | | | |
| | 18.100 | 200.00 | | | | | | | 80 | 1143 | 37258 | 461257 | 287050 | | | | |
| *29 | std0001 | XYA | | | | | | | | | | | | 18.075 | 18.117 | 18.167 | 18.17 |
| | 18.117 | 50.00 | | | | | | | | | | | | 59412 | 112086 | 54548 | 383 |
| *30 | std0001 | 3OMD | | | | 21.575 | 21.608 | 21.608 | 21.600 | 21.592 | | | | | | | |
| | 21.608 | 10.00 | | | | 58 | 2183 | 11535 | 2175 | 143 | | | | | | | |
| *31 | std0001 | 5HTOL | 22.167 | 22.200 | 22.200 | 22.217 | | | | | | | | | | | |
| | 22.200 | 10.00 | 47 | 1668 | 19401 | 1931 | | | | | | | | | | | |
| *32 | std0001 | HVA | | | | 23.925 | 23.925 | 23.917 | 23.917 | 23.925 | 23.925 | 23.950 | | | | | |
| | 23.917 | 200.00 | | | | 225 | 13994 | 237705 | 163964 | 47372 | 8852 | 2293 | | | | | |
| *33 | std0001 | XYI | | | | | | | | | | | 24.267 | 24.258 | 24.283 | 24.300 | 24.308 | 24.3 |
| | 24.258 | 100.00 | | | | | | | | | | | 101620 | 108998 | 55261 | 39928 | 21718 | 466 |
| 34 | std0001 | TTRA | | | | | | | | | 24.475 | 24.492 | 24.517 | | | | |
| | 24.492 | 80.00 | | | | | | | | | 2368 | 28935 | 5390 | | | | |
| 35 | std0001 | 5HT | 30.742 | 30.783 | 30.792 | | | | | | | | | | | | |
| | 30.792 | 10.00 | 231 | 6955 | 9627 | | | | | | | | | | | | |
| 36 | std0001 | 3HT | | | | 31.967 | 31.975 | 31.975 | 31.967 | 31.975 | | | | | | | |
| | 31.975 | 100.00 | | | | 143 | 11501 | 33026 | 2425 | 171 | | | | | | | |
| *37 | std0001 | TPOL | | | | | | | 41.500 | 41.475 | 41.458 | 41.467 | 41.492 | | | | |
| | 41.467 | 10.00 | | | | | | | 375 | 2006 | 7109 | 9905 | 4810 | | | | |
| *38 | std0001 | TRP | | | | 45.350 | 45.342 | 45.300 | 45.300 | 45.275 | 45.292 | 45.325 | 45.350 | 45.350 | | | |
| | 45.292 | 700.00 | | | | 615 | 8371 | 23358 | 248497 | 967515 | 2056846 | 1090677 | 187013 | 109610 | | | |

## Table 3. Unknown Pool Peak Matching.

Table shows dominant or maximum channel height and retention times of unknown peaks in the pool CSF with default concentrations set at 100. The peaks are referenced to XAN and Tyrosine, concentration 1000; HVA, concentration 200; Tryptophan, concentration 700.

The table shows the match of the seventh pool in the study to the nineteenth pool in the study and this represents typical drift over a two week period.

*   Indicate variables used in regression analysis.

8

## Table 3

Sample: PCOL17
Standard:
Table: PCOL19A
 Study: C3F19A
# Compounds Identified: 22
# Known Compounds Not Found: 0
# Unknown Peak Clusters: 745

Compounds Identified

| | Compound | Conc | RT | RT Error | Height | Ratio Accuracy | |
|---|---|---|---|---|---|---|---|
| | | | | | | 7/6 | |
| * | p01 | 104.413971 | 2.617 | 0.100 | 167971 | 0.999 | |
| | | | | | | 10/11 | 12/11 |
| | XAN | 973.750732 | 2.900 | 0.117 | 756026 | 0.861 | 0.959 |
| | | | | | | 15/16 | |
| * | p02 | 101.869156 | 3.108 | 0.125 | 208822 | 0.666 | |
| | p03 | 131.687439 | 5.733 | 0.275 | 2747 | | |
| | | | | | | 13/14 | 15/14 |
| | p04 | 106.228569 | 8.242 | 0.325 | 142085 | 0.977 | 0.891 |
| | | | | | | 9/10 | 11/10 |
| | TYR | 979.260254 | 13.567 | 0.692 | 1375195 | 0.987 | 0.977 |
| | | | | | | 13/14 | |
| * | P05 | 99.553802 | 14.633 | 0.583 | 52515 | 0.949 | |
| | | | | | | 9/10 | 11/10 |
| | P09 | 85.990860 | 15.117 | 0.650 | 7632 | 0.850 | 0.923 |
| | | | | | | 14/15 | |
| * | P07 | 92.967072 | 15.275 | 0.600 | 55664 | 0.951 | |
| | | | | | | 10/11 | |
| | P08 | 96.659180 | 16.133 | 0.417 | 29579 | 0.976 | |
| | | | | | | 9/10 | |
| * | P10 | 96.603462 | 21.875 | 0.942 | 42516 | 0.949 | |
| | | | | | | 5/6 | 7/6 |
| | HVA | 195.139450 | 22.475 | 0.800 | 30508 | 0.987 | 0.921 |
| | | | | | | 16/15 | |
| | P11 | 102.689194 | 24.892 | 0.717 | 16223 | 0.892 | |
| | | | | | | 9/10 | |
| | P12 | 91.103188 | 25.150 | 0.850 | 10652 | 0.951 | |
| * | p18 | 98.121086 | 26.850 | 1.308 | 470 | | |
| * | p19 | 121.804512 | 27.192 | 1.142 | 224 | | |
| | | | | | | 12/13 | |
| | P13 | 95.415916 | 29.158 | 0.883 | 53718 | 0.960 | |
| | | | | | | 13/14 | |
| | P14 | 97.315338 | 29.542 | 1.108 | 24489 | 0.969 | |
| | | | | | | 9/10 | |
| | P15 | 94.104630 | 29.642 | 1.100 | 10631 | 0.916 | |
| | | | | | | 10/11 | |
| | P16 | 83.826569 | 32.425 | 0.767 | 2937 | 0.761 | |
| | | | | | | 13/14 | |
| | P17 | 96.782722 | 37.992 | 1.133 | 22737 | 0.958 | |
| | | | | | | 9/10 | 11/10 |
| | TRP | 574.284887 | 42.150 | 1.583 | 277933 | 0.979 | 0.963 |

The analysis records were linked by a unique identifier to clinical data of clinical diagnosis, diagnostic criteria, age, pharmaceutical history, sex and race. Pools analyzed as samples against standards for known values were utilized to assess the precision of known compound values in the data base. Standards analyzed against sequential standards were used as a measure of instrumental performance and pools analyzed against sequential pools were utilized as a measure of the precision of unknown peaks.

Validation of the Data

Control standards analyzed against sequential control standards yielded precision values ranging from ±1%-±4% CV with no outlying values. Pools analyzed as samples gave precision values ranging from ±2-±7% CV for compounds present at the 0.5 ng/mL level or greater and typically ±25-30% for compounds present at 2X the detection limit of 0.02-0.03 ng/mL (e.g. 5HT, EPI). Pools analyzed against sequential pools for unknowns gave values of ±3-±15% coefficient of variation. Typically, the coefficient of variation of the pools was 5-25 fold less than the coefficient of variation of analytes in a group of samples. Essentially, the contribution of assay variability to the results is minimal.

The data base, upon completion, contained 280 samples by 57 analytes (17,000 records). Of these, 163 were null either because no peaks were detected at the sensitivity limits of the assay, or because a signal detected did not meet the qualitative criteria for purity.

Regression Analysis

Linear regression analysis and stepwise regression analysis were used in a preliminary evaluation of the data. Both raw and mean corrected data was evaluated.

Regression comparison of the AD group (61) vs. controls (60) setting AD=1 and C=0 gave a categorical separation regression equation with an S (standard error of estimate) value=0.39 and p=.0041 for 27 of the most significant known compound variables identified in stepwise regression (labeled with asterisks in Table 2). Inclusion of 7 of the most significant variables (labeled with asterisks in Table 3) from the pool analyzed unknown peak data base gave values of S=0.382 and p=0.0037. Assuming a clinical diagnostic error rate in the order of 10%, seven AD samples with regression calculated values (from -1.2 to 0.01) were removed from the calculation. The regression characteristics were then S=0.352 and p=0.0031.

Regression of the AD group with AD=1 vs. all others (219)=0 for the same variable group yielded an equation with S=0.481 and p=0.0013.

Observations: Although the AD group is separated from mother groups with a high degree of probability, there is too high a degree of overlap for a simple linear regression algorithm to accurately categorize an individual sample.

Cluster Analysis Procedures

Cluster analysis procedures using nearest neighbor and furthest neighbor approaches were applied to the data base. With both these approaches, the AD group tended to cluster, but controls were scattered relatively evenly, both outside and inside the AD region. Thus, the cluster analysis approach is not suitable as a categorization tool for this type of data.

Observations: The behavior of the data under cluster analysis protocols, and the observations that the standard deviations of compound values and of precursor/product ratios across metabolic pathways within a disorder group are smaller than within control groups is consistent with the hypothesis that the biochemical response of controls or normal individuals is more chaotic than that of disordered individuals.

Frequency Distribution Probability Analysis

The observations on the nature of the data distributions coupled with the technical ability to run large numbers of samples and variables offers an approach to categorization based on differences in the frequency distributions of variables in different disorder categories. This approach relies on basic probability considerations without any assumptions on the shape of a distribution curve or linearity of relationships.

The simplest question that I have investigated for the preliminary data base is that given an unknown sample, what is the probability (p) that that sample belongs in one group and not another.

For one variable, the question takes the form:

$$P = \frac{f(V_1)_A}{f(V_1)_A + f(V_1)_B}$$

where $F(V_n)_A$ or $f(V_n)_B$ = the frequency with which an unknown sample value ($V_n$) occurs in category A or category B. For multiple compounds, the expression expands:

$$P = \frac{f(V_1)_A \cdot f(V_2)_A \ldots f(V_n)_A}{f(V_1)_A \cdot f(V)_A \ldots f(V_n)_A + f(V_1)_B \cdot f(V_1)_B \ldots f(V_n)_B}$$

If all frequencies are the same, the P value is 0.5 or a 50/50 chance that the unknown sample is A and not B. A positive answer compresses the expression to a 1 and a negative answer to 0.

Like cluster procedures and unlike regression, the use of the algorithm is independent of the number of variables used.

Implementation of the Procedure:

The implementation of the procedure is by the following steps:

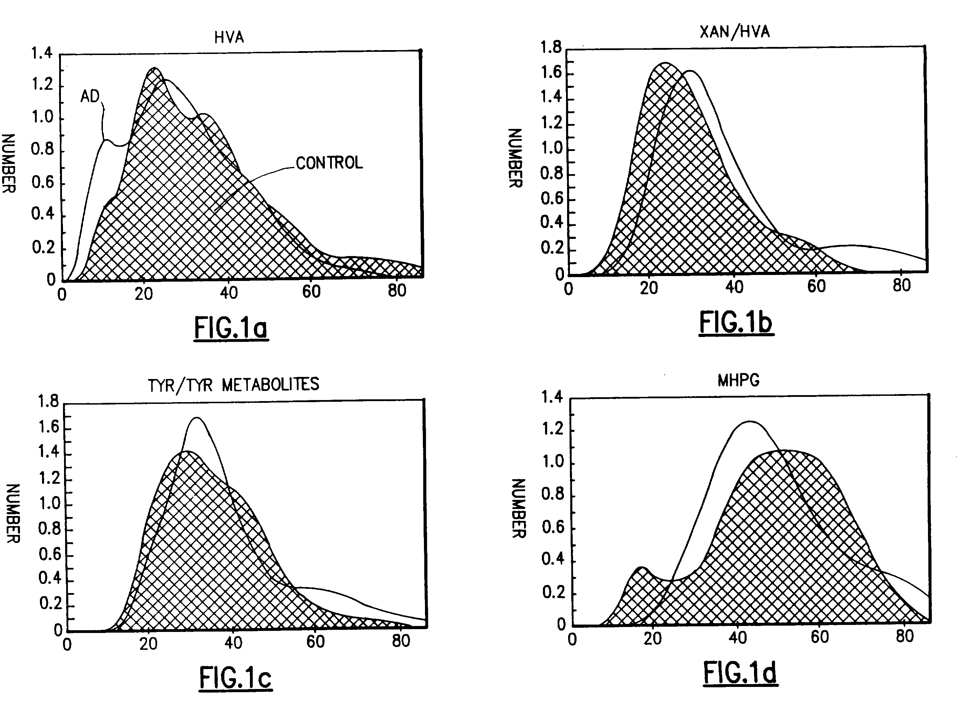

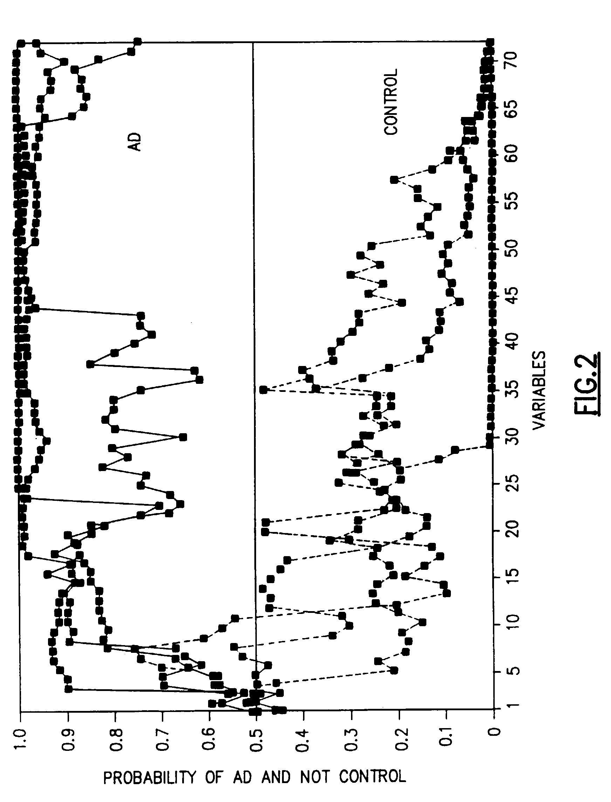

1. Frequency distributions (shown in Figs. 1a-1d) were created by using a smoothing algorithm based on a 3 point polynomial expansion function that treats each point in the sparse data distribution with equal weighting as the means of a distribution with a width at half height proportional to its value. The use of smoothing functions is a necessary assumption until the n of samples in a particular category reaches approximately 300-400. The procedure used was to divide all data in the categories by the maximum value among categories X 85, apply the polynomial expression algorithm, and normalize the data distributions for the number of samples in each category. The frequency distributions in each category are then organized into look up tables for each variable (Table 4) is then inserted into the look up table. Individual values are divided by the range value X 85 and the frequencies for each variable for category A and B are sequentially calculated in the algorithm after subtracting the effect of that sample from the frequency table. The effect of a sequential calculation across a group of variables listed and described in Table 6 is shown in Figure 2 for 3 AD and 3 C cases from a group of 61 AD and 44 controls. One of the major features of the algorithm is that no single variable predominates as a differentiator among a large group of samples.

TABLE 4. Distribution Table.

The table shows frequency distribution intervals 1-85 for two of the 71 variables in a look up table initially used in scoring ad and control samples.

The tables are obtained by dividing all data by the highest value for the combined data base times 85.

The raw distribution is then smoothed by a polynominal expansion which treats each value with equal weighting as the mean of a distribution whose scatter increases with the value.

The overall distributions of groups are normalized to the same area.

# DISTRIBUTION TABLE

| | HVA | | | MHPG | |
|---|---|---|---|---|---|
| | AD | CONTROL | | AD | CONTROL |
| 1 | 0.010377 | 0.000205 | 1 | 1.2E-10 | 0.000000 |
| 2 | 0.045259 | 0.001702 | 2 | 0.000000 | 0.000003 |
| 3 | 0.126131 | 0.007945 | 3 | 0.000000 | 0.000026 |
| 4 | 0.242295 | 0.025793 | 4 | 0.000000 | 0.000140 |
| 5 | 0.379120 | 0.064431 | 5 | 0.000000 | 0.000576 |
| 6 | 0.503888 | 0.129976 | 6 | 0.000001 | 0.001932 |
| ⋮ | ⋮ | ⋮ | ⋮ | ⋮ | ⋮ |
| 38 | 0.798418 | 0.938551 | 38 | 1.117459 | 0.742461 |
| 39 | 0.761982 | 0.889793 | 39 | 1.160736 | 0.801194 |
| 40 | 0.730955 | 0.836392 | 40 | 1.196534 | 0.855713 |
| ⋮ | ⋮ | ⋮ | ⋮ | ⋮ | ⋮ |
| 83 | 0.006846 | 0.088021 | 83 | 0.225452 | 0.069938 |
| 84 | 0.005088 | 0.081885 | 84 | 0.208132 | 0.054130 |
| 85 | 0.003699 | 0.075893 | 85 | 0.190170 | 0.011177 |

2. A sample record such as that shown in Table 5

## TABLE 5. DATA CASE RECORD.

The data case record is spooled from the Oracle data base. In the example shown, the query requested biochemical data, the diagnosis "control" C and the I.D. "AT0022".

The distribute range is created from the record by dividing each value by the maximum range value times 85 to indicate the look up range for the freqency of occurrance of the compound in the AD and C distributions.

Table 5

# DATACASE RECORD

| | C AT0022 | DISTRIBUT RANGE |
|---|---|---|
| MT3 | 0.018080 | 1 |
| OHAN3 | 0.018080 | 3 |
| OHKY3 | 0.090402 | 4 |
| 3OMD | 1.970763 | 44 |
| ⋮ | ⋮ | ⋮ |
| HVA | 58.03809 | 56 |
| MHPG | 8.714754 | 62 |
| ⋮ | ⋮ | ⋮ |
| 12 | 2.911604 | 22 |
| PO1 | 57.25159 | 9 |
| PO2 | 135.9827 | 49 |
| PO3 | 1.808040 | 1 |
| ⋮ | ⋮ | ⋮ |

TABLE 6. DESCRIPTION OF SCORING VARIABLES.

The table shows the arbitrary sequence in which 71 variables were applied to the initial scoring of AD vs. C (See Figure 2).

The order of scoring has no effect on the final outcome. Acetaminophen which was included in the assays was not used as a scoring variable.

The table also contains the names of the known compounds assayed and the probable moities in the unknown peaks inferred from the chromatographic/electrochemical data and in vitro studies. Pathway ratios one to twelve are calculated as molar ratios.

## Table 6

### DESCRIPTION OF SCORING VARIABLES

| Variable Number | Abbreviation | Name or Possible Characteristic |
|---|---|---|
| 1 | 3MT | 3 - Methoxytyramine |
| 2 | 30HAN | 3 - Hydroxyanthranilic Acid |
| 3 | 30HKY | 3 - Hydroxykynurenine |
| 4 | 30MD | 3 - 0 - Methyldopa |
| 5 | 4HBAC | 4 - Hydroxybenzoic Acid |
| 6 | 4HPAC | 4 - Hydroxyphenylacetic Acid |
| 7 | 4HPLA | 4 - Hydroxyphenyllactic Acid |
| 8 | 5HIAA | 5 - Hydroxyindoleacetic Acid |
| 9 | 5HT | 5 - Serotonin |
| 10 | 5HTOL | 5 - Hydroxytryptophol |
| 11 | 5HTP | 5 - Hydroxytryptophan |
| 12 | A | Adenine |
| | AM | Acetaminophen |
| 13 | ASC | Ascorbic Acid |
| 14 | CYS | Cysteine |
| 15 | DA | Dopamine |
| 16 | DOPAC | Dihydroxyphenylacetic Acid |
| 17 | EPI | Epinephrine |
| 18 | G | Guanine |
| 19 | GR | Guanosine |
| 20 | GSH | Glutathione (reduced) |
| 21 | HGA | Homogentisic Acid |
| 22 | HVA | Homovanillic Acid |
| 23 | HX | Hypoxanthine |
| 24 | KYA | Kynurenic Acid |
| 25 | KYN | Kynurenine |
| 26 | LD | L-Dopa |
| 27 | MET | Methionine |
| 28 | MHPG | Methoxy-Hydroxyphenyl Glycol |
| 29 | NE | Norepinephrine |
| 30 | NMN | Normetanephrine |
| 31 | TPOL | Tryptophol |
| 32 | TRP | Tryptophan |
| 33 | TYR | Tyrosine |
| 34 | TYRA | Tyramine |
| 35 | URIC | Uric Acid |
| 36 | VMA | Vanillylmandelic Acid |

Table 6 (Cont.)

| Variable Number | Abbreviation | Name or Possible Characteristic |
|---|---|---|
| 37 | XAN | Xanthine |
| 38 | P01 | Methoxyhydroxybenzene - |
| 39 | P02 | Cysteine or Methionine Di Peptide |
| 40 | P03 | Catechol - |
| 41 | P04 | Catechol - |
| 42 | P05 | Cysteine Condensation or Peptide |
| 43 | P07 | Cysteine Condensation or Peptide |
| 44 | P08 | TRP or TYR Peptide |
| 45 | P09 | TRP or TYR Peptide |
| 46 | P10 | Indole - |
| 47 | P11 | Indole - |
| 48 | P12 | Methoxybenzene - |
| 49 | P13 | Indole - |
| 50 | P14 | Hydroxybenzene - |
| 51 | P15 | TYR or TRP Peptide |
| 52 | P16 | TYR or TRP Peptide |
| 53 | P17 | Indole - |
| 54 | P18 | Hydroxyindole - |
| 55 | P19 | Hydroxyindole - |
| 56 | HVA_A | Oxidative of Backwave of HVA |
| 57 | TPR_A | Oxidative of Backwave of TRP |
| 58 | TYR_A | Oxidative of Backwave of TYR |
| 59 | XAN_A | Oxidative of Backwave of XAN |
| 60 | 1 | TRP/5HIAA + 5HTOL + 5HT + 5HTP |
| 61 | 2 | TRP/ OHAN + KYN |
| 62 | 3 | TRP/KYN + HTP + HTOL + 5HIAA + 5HT |
| 63 | 4 | HTP/5HIAA |
| 64 | 5 | 5HIAA/5HTOL |
| 65 | 6 | KYN/OHKY |
| 66 | 7 | HVA/5HIAA |
| 67 | 8 | TYR/HPLA |
| 68 | 9 | TYR/HVA + LD + E + NE + MHPG + DA + 3MT + NMN |
| 69 | 10 | HVA/MHPG |
| 70 | 11 | TYR/HPLA + HVA + LD + E + NE + MHPG + DA + 3MT + NMN |
| 71 | 12 | XAN/HVA |

Testing the Algorithm on AD vs. Controls

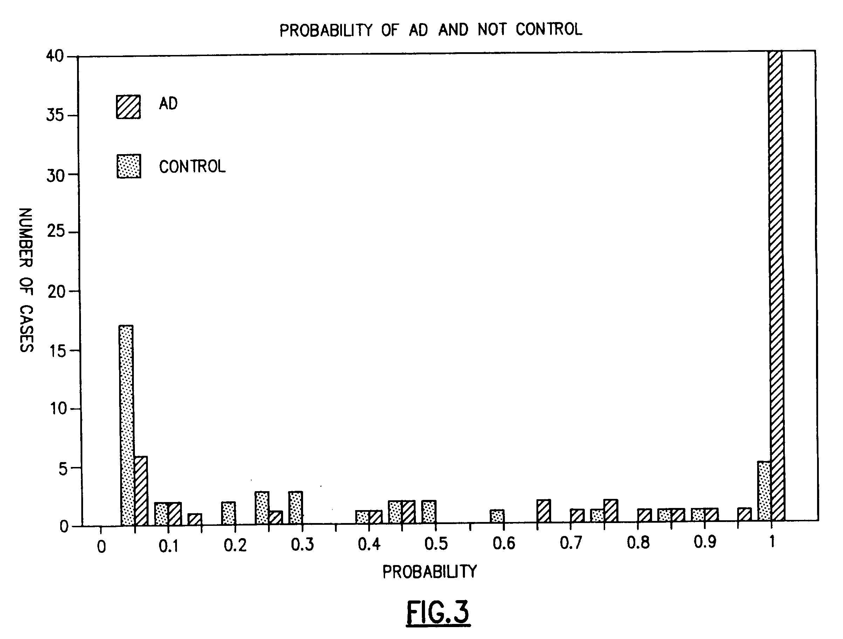

For an initial test, conditions were set up such that each individual sample was evaluated as if the data base were set up without its contribution. The results of the initial scoring are shown in Fig. 3. The scoring of five of the 61 AD cases as controls ($p$=less than 0.01 that the sample is an AD and not a control) is not surprising given the probable diagnostic error rate in AD. The scoring of 4 of the controls as AD are of concern.

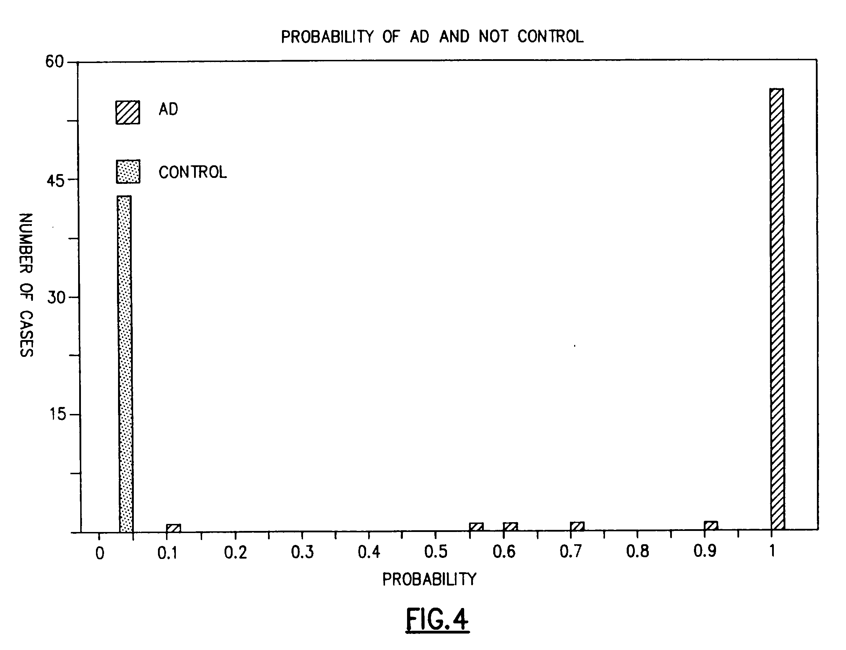

One possible explanation is that the AD data base is in effect contaminated by five cases that clearly do not match the overall AD profile and are probably not AD. When these five samples are removed from the AD data base and all samples, including the 5 removed, are scored, the control and AD groups are uniquely separated as shown in Fig. 4. The five samples that were removed from the AD scoring data group distribute in an equivocal region from 0.1 to 0.9.

In subsequent application of the procedure and algorithm to AD samples vs. all other samples (PD, SC, HD, PSP and C) in the data base asking the question is this sample in the AD distribution and not in the distribution of all others yielded similar results scoring AD samples with p values = 0.98 or greater. The distribution of scores of all others was scattered from 0.001 to .8 including the 5 AD samples which previously scored in this region vs. controls.

Testing PD vs. Controls and PD vs. AD

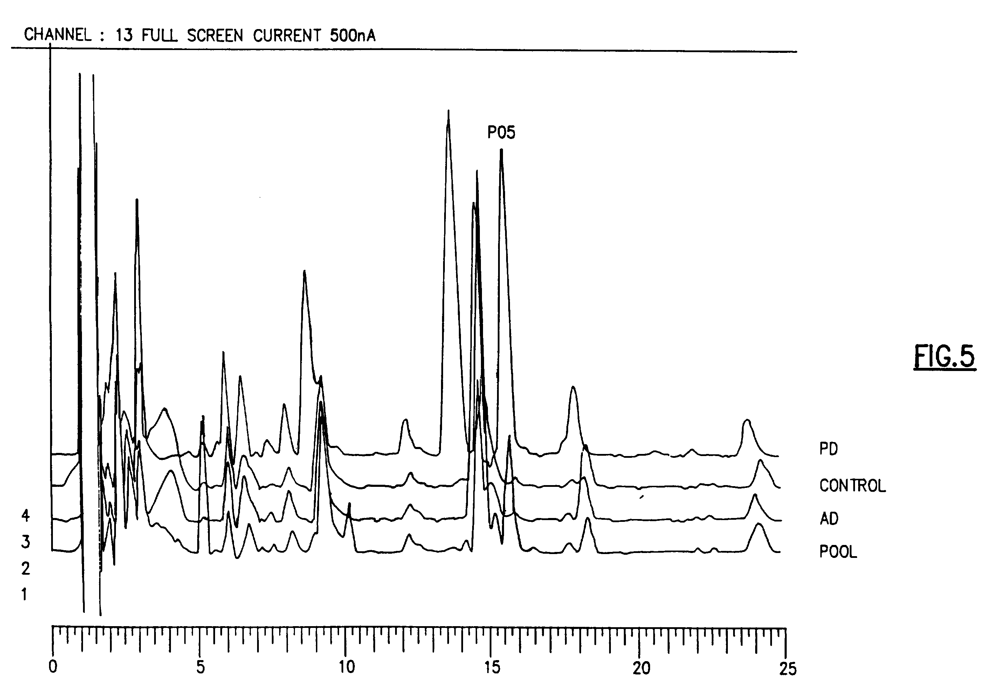

Eighteen unknown peaks were initially selected for assay in all samples because of differences among preliminary pools of AD, PC, C and SC. Of these, six occurred only in one sample of the group and 11 had distributions of only slight differentiation capability. One peak, designated PO5 shown in Figure 5, occurred predominantly in the PD group. Otherwise, values above 100% of pool values were seen in only six of the AD group. The mean values of PO5 and S. D. relative to a pool value of 100 were PD 415 ± 200, Control 20 ± 15, AD 30 ± 80, and SC 28 ± 17. This one variable utilized alone in the distributin p scoring algorithm separates PD from controls with PD values from 0.4 - 0.999 and C values from 0.51 - 0.001. With all variables, the PD values were all greater than 0.994 and C values less than 0.003.

Scoring AD and not PD without PO5 separated 58 PD samples with scores of less than 0.02 and two with scores of 0.14 and 0.18. 55 AD samples scored above 0.98 with six in the region between 0.25 and 0.76, including the 5 cases which initially scored as controls. Including PO5 in the scoring, all PD samples scored below 0.001. 57 of the AD samples scored above 0.98 and 4 of the initial 5 that matched the control group scored near the PD samples from 0.11 to 0.32.

Example II

Pathological Changes in the Olfactory System in Alzheimer's Disease

In Alzheimer's Disease a number of studies have shown that the structures of the limbic system, including entorhinal cortex, hippocampal formation, basal forebrain and the amygdala are the most severely affected areas of the brain. Since the amygdala, entorhinal cortex and uncal hippocampus are strongly related to olfactory input (Pearson et al, 1985)[1] suggested that the olfactory pathways may be the initial site of pathology in the disease. In fact some authors have suggested that inhaled molecules, such as aluminosilicates, could contribute to the etiology of the disease (Roberts, 1987).[2]

The olfactory nerve cells in the olfactory epithelium project through the cribiform plate to the olfactory bulb (Kosel et al, 1981)[3] Primary olfactory fibers synapse in olfactory glomeruli with descending dendrites of mitral and tufted cells; which are the primary output neurons of the bulb. Axons of mitral and tufted cells enter the olfactory tract and provide input to the anterior olfactory nucleus, as well as to the central projections of the olfactory system. The anterior olfactory nucleus, located in the center of the bulb, gives rise to a recurrent collateral to the bulb and to a crossed projection to the anterior commissure. The olfactory tract passes through the anterior perforated substance and projects to prepiriform cortex, corticomedial nuclei of the amygdala, entorhinal and perirhinal cortices, inferior surface of the frontal lobe, insula, temporal pole and basal forebrain (Haberty et al, 1977).[4]

Several lines of evidence have shown that the olfactory system is affected in Alzheimer's Disease. Several groups have shown that Alzheimer's Disease patients show deficits in olfactory recognition (Doty et al, 1987).[5] A study which examined patients with very mild Alzheimer's Disease showed impairment on a task of identification of odors, however olfactory thresholds were normal (Koss et al, 1988).[6] Pathologic studies have recently documented changes in the primary olfactory receptor neurons in Alzheimer's Disease. Talamo et al, 1976[7] reported nasal epithelium from Alzheimer's Disease patients showed increased immunoreactivity for phosphorylated neurofilaments, as well as Tau and Alz-50 positive neurites. These neurons however did not contain neurofibrillary tangles. Neurite formation in the olfactory epithelium did correlate with numbers of neurofibrillary tangles and senile plaques in the hippocampus of the Alzheimer's Disease brains. The possibility of using biopsy of nasal epithelium as a clinical marker of Alzheimer's Disease during life was suggested.

Pathological studies of the olfactory bulb in Alzheimer's Disease have shown consistent changes. Esiri and Wilcock, (1984)[8] found neurofibrillary tangles in the olfactory bulb in tufted cells, outer granule cells, and in the anterior olfactory nucleus. Hyman and colleagues (1991)[9] has examined 10 control and 10 Alzheimer's Disease olfactory bulbs. Large numbers of neurofibrillary tangles were consistently found in the Alzheimer's Disease anterior olfactory nucleus, with only small numbers in the mitral and tufted cells. This is consistent with other primary sensory systems in Alzheimer's Disease in which higher order association areas show more severe pathology than primary sensory areas.

The olfactory epithelial neurons and olfactory bulb have been shown to contain very high levels of carnosin (B-alanyl-L-histidine), and several studies have shown that carnosine is released in the olfactory bulb in response to peripheral inputs (Macrides et al, 1983).[10] The external tufted cells in the olfactory bulb contain dopamine, substance P or both. The deeper tufted cells may use excitatory amino acids. Periglomerular cells contain GABA, enkephalin or dopamine. The olfactory bulb receives a very strong centrifugal projection including enkephalin, vasoactive intestinal polypeptide, LHRH, somatostatin and substance P fibers. A cholinergic projection originates mainly in the ventral nucleus of the diagonal band. Serotonergic projections originate from the dorsal raphe nuclei, while there is a noradrenergic projection from the locus ceruleus. The olfactory bulbs therefore receive a rich and variegated neurochemical input, including projections from the cholinergic, noradrenergic and serotonergic nuclei which are known to be affected in Alzheimer's Disease (Macrides et al, 1983).[11]

Several factors relating to the suitability of using nasal secretions for neurochemical analysis have been examined. These include sample acquisition, potential effects of bacterial or viral infection, the specific compounds which can be identified, and means for normalizing the data to compensate for sampling variability and sample size.

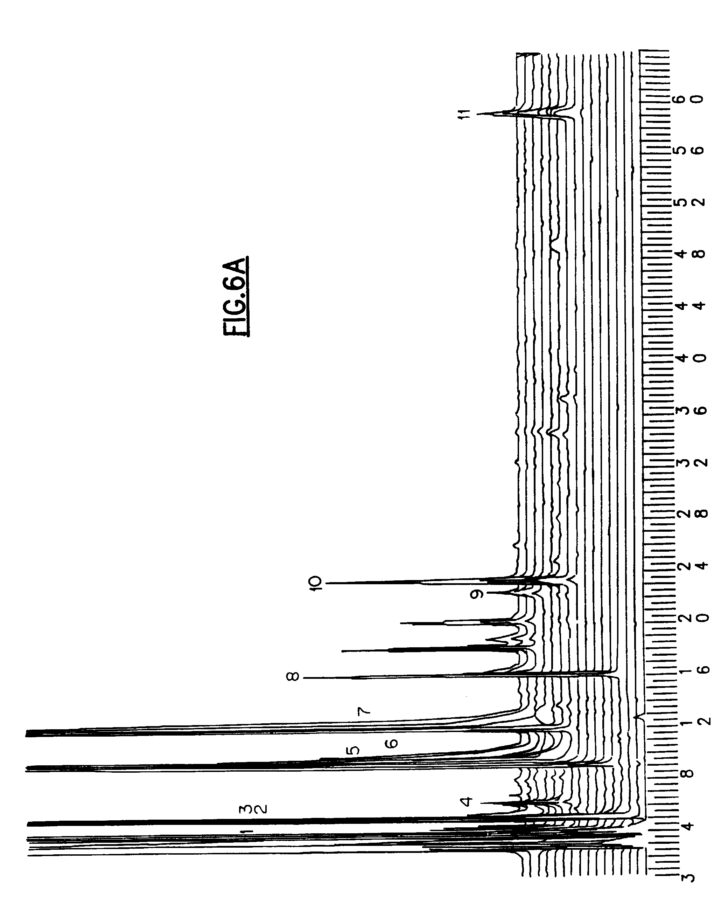

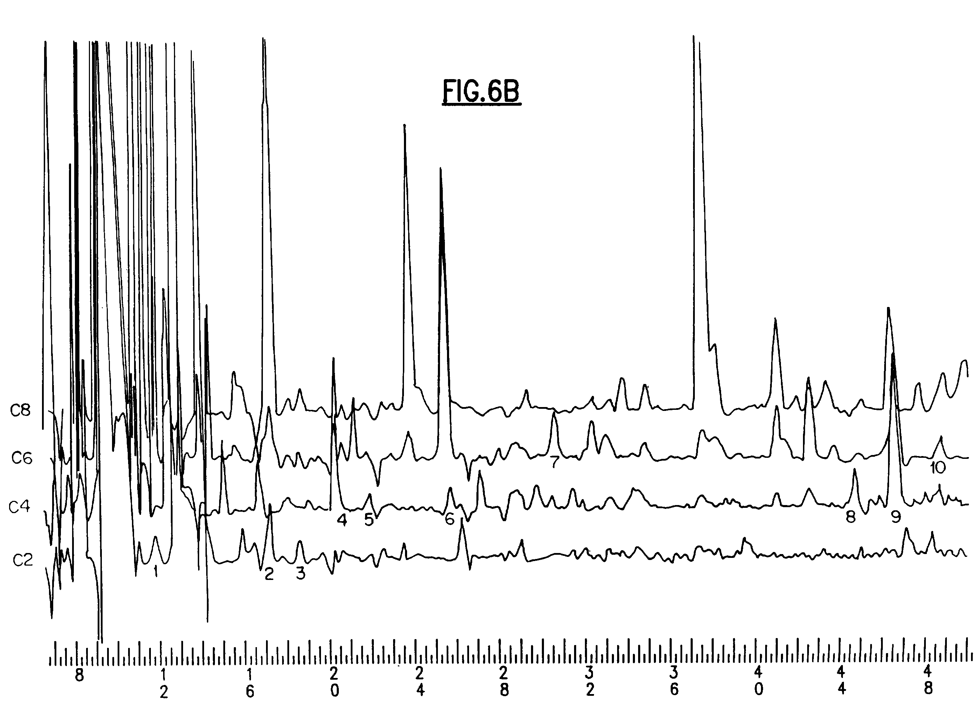

Following the electrochemical analysis procedure above described for CSF all the compounds listed in Table 2 have been identified using nasal secretion samples although the concentration profiles are significantly different than for CSF. (See typical patterns in Figures 6A and 6B.) Notably, dihydroxyphenylacetic acid (DOPAC) is present in greater concentrations than homovanillic acid (HVA) and serotonin (5HT) is relatively equivalent to 5-hydroxindoleacetic acid (5HIAA). Also notably different is the complexity of the region of small di-and tripeptides with retention times of 30-45 min on channels 10-15. Essentially, the nasal mucosa appear to contain representative compounds from all metabolic pathways that occur in the neuronal projections to the olfactory bulb.

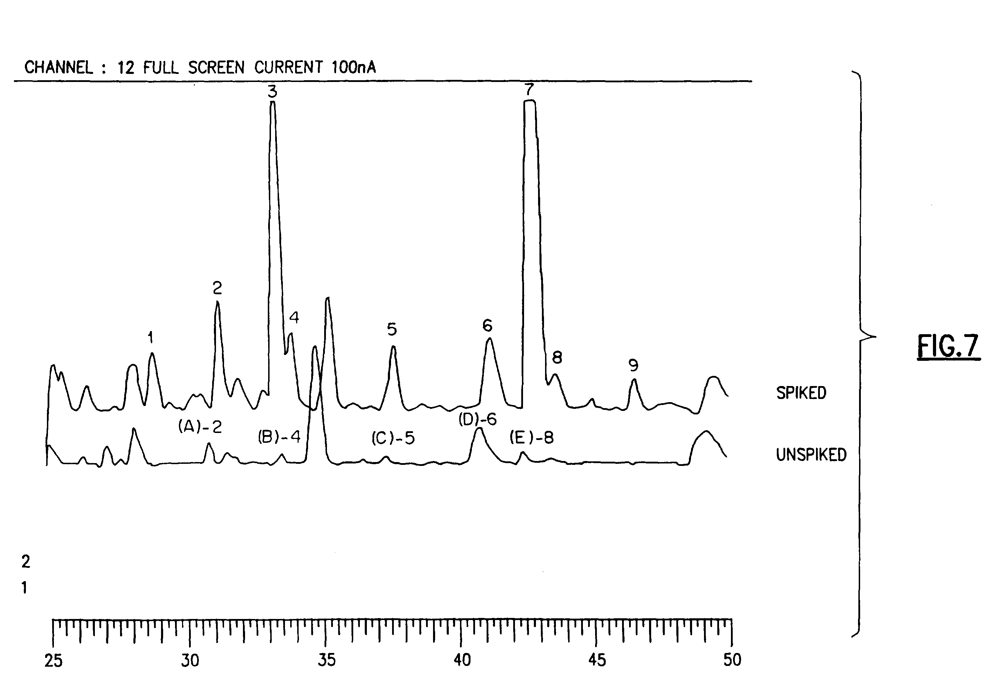

Two characteristics make nasal secretions attractive for neurochemical analysis. The secretions are close to the site or origin of the compounds being measured and they are in a strong reducing environment. Oxidation potential measurements of saline suspensions of nasal secretions with platinum vs. Ag/Ag/Cl yield values from -0.230 to -0.300 mV compared to lumbar CSF values of -0.050 to -0.170. The reducing character of the tissue is consistent with observation of a large number of easily oxidized peaks on the lower channels of the chromatographic pattern. The reducing character of the sample makes it highly attractive for clinical use because of the stability inferred for the target compounds. Indeed preliminary studies indicate no change in 5-hydroxyindoleacetic acid or homovanillic acid over 24 hours in perchloric acid extracts at room temperature. From the number of peaks in the 35-50 min region, the nasal mucosa also appear to be a stable matrix for small peptides, including fragments of $\beta$-amyloid peptide. This hypothesis was tested by incubating $\beta$-amyloid in saline suspensions of nasal mucosa and observing the development over 6 hours of electroactive peaks, five of which corresponded to original peaks in the suspension as shown in Figure 7.

Sample Acquisition Preparation and Normalization

Samples typically weigh approximately 100 mg. A preliminary evaluation of swabs taken from high in the nostril, approximately 2 cm, vs. a wipe of the nares at approximately 0.5 cm, indicate that all species of interest are present lower in the nose, but at significantly reduced concentrations overall. In an initial study from an n of 4 there were no significant changes in the ratio of DOPAC/HVA or in tryptophan (TRP) to kynurenine (KYN), but the ratio of 5HIAA/5HT increased, consistent with reduced concentrations of 5HT in swabs obtained lower in the nose. Similarly, on an n of 4, a relatively aggressive scrubbing the swab yields a pattern with higher concentrations than a more gentle scrub, but without significant changes in overall pattern.

Swabs were extracted by cutting the tip and placing it in 300µl of 0.1 M $HClO_4$, they ere vortexed for 1 min. centrifuged and then reextracted in another 300 1 of 0.1 M $HClO_4$. Further sequential extractions did not result in any improvement in recoveries. Following centrifugation the pellets are combined, and can be extracted with 40:60 acetonitrilehexane for analysis of ubiquinones and larger peptides.

Several brands of cotton swab were evaluated for blank effects. Of these, plastic handled Johnson and Johnson (TM) swabs proved clean enough to be used as received. Paper composite and wooden handles of various types showed a number of small peaks that have the potential to interfere with the target compounds of interest.

The selection of appropriate divisors or normalizers of the data is a major consideration since sample size is not controllable. This issue was investigated in a preliminary study of 6 individuals from whose left and right nostril swabs were obtained. From the initial data analysis, xanthine (XAN), uric acid (UA), tyrosine (TYR), DOPAC, HVA, TRP, KYN, 5HIAA and 5HT have been measured. The 6 left and right individual samples as duplicate pairs coefficient of variation were from ±60 - ±80%. When all data was divided by xanthine, the variations were ±15 - ±30%. When precursor product ratios were used, the variations were further lowered (DOPAC/HVA mean 1.4 ± 7%; TRP/KYN mean 2.6 ± 14%; and 5HT/5HIAA mean 1.1± 13%)

Possible Effects of Bacterial and Viral Infections

Two available cultures of Pseudomonas and Staphylococcus were evaluated to look for possible interferences from bacterial infection. Aliquots of approximately 10 mg wet weight of cultured bacteria isolated by centrifugation and washing were sonified in 200 L of 0.1 M $HClO_4$, centrifuged and the supernatant analyzed. Responses for XAN, KYN, TYR, TRP, guanosine and hypoxanthine were observed of approximately 50-100% of the response of a typical nasal swab. No dopaminergic, serotonergic, or noradrenergic metabolites were observed. Consequently, it is thought that bacterial contamination would have to be quite massive, constituting over 10% of a typical sample to have a 10% or greater effect on the measurements.

In the initial study three of the six subjects had colds and there were no significant differences between them and unaffected individuals. There was a slight, but not statistically significant increase in KYN/XAN and a decrease in TRP/KYN ratios.

It thus appears that nasal secretions may advantageously be employed as samples for neurochemical analysis using electrochemical detection techniques. Similar results were found using platelets.

Moreover, the invention advantageously may be employed for diagnosing a disease condition at an early stage, i.e. before observable physical manifestations. For example, in the case of Alzheimer's Disease, the exact etiology is unknown. However, there is strong evidence that genetic factors play a role (St. George-Hyslop et al, 1990).[12] The means by which a genetic defect contributes to the pathologic features of the illness is unclear. A major feature of the pathology is the accumulation of the β-amyloid protein in senile plaques, blood vessels, skin and other peripheral tissues (Joachim et al., 1988).[13]

By correlating changes in neurochemical markers with changes in accumulation of the β-amyloide protein as the Alzheimer's Disease progresses, it is possible to provide an early diagnosis for Alzheimer's Disease. Also, identification of neurochemical markers for Alzheimer's Disease may provide a basis for prevention and/or treatment, i.e. by identifying precursors, progression of the disease may be slowed, stopped or even reversed.

The invention has been described for use in diagnosing Alzheimer's Disease. It will be understood, however, that the invention advantageously may be used to diagnose and characterize other neurological, degenerative or defective disorders such as Huntington's Disease, Parkinson's Disease, schizophrenia, progressive supernuclear palsy, amyotrophic lateral sclerosis and senile dementias. The invention also advantageously may be used to classify and diagnose tumors, carcinomas, cardiovascular abnormalities and other disorders, or for selection of therapy based on categories of known successful vs. unsuccessful outcomes. Moreover, both treatment protocols and new pharmaceuticals may be evaluated.

1. Pearson R.C.A., Esiri M.M., Hiorns R.W., Wilcock G.K. Powell T.P.S. Anatomical correlates of the distribution of the pathological changes in the neocortex in Alzheimer's disease. Proc. Natl. Acad. Sci. 82: 4531-45 24, 1985.

2. Roberts, E. Alzheimer's disease may begin in the nose and may be caused by aluminosilicates. Neurobiol. Aging. 7:561-567, 1986; Shipley M.T. Transport of molecules from nose to brain; transneuronal anterograde and retrograde labeling in the rat olfactory system by wheat germ agglutinin-horseradish peroxidase applied to the nasal epithelium. Brain Res. Bull. 15: 129-142, 1985; Peri D.P., Good P.F. Uptake of aluminum into central nervous system along nasal-olfactory pathways. Lancet 1: 1028, 1987.

3. Kosel K.C., Van Hoesen G.W., West J.R. Olfactory bulb projections to the parahippocampal area of the rat. J. Comp. Neurol. 198: 467-482, 1981.

4. Haberty L.B., Price J.L. The axonal projection patterns of the mitral and tufted cells in the olfactory bulb in the rat. Brain Res. 129: 152-157, 1977.

5. Doty R.L., Reys P.F., Gregor T. Presence of both odor identification and detection deficits in Alzheimer's Disease. Brain Res. Bull. 18: 597-600, 1987; Green J.E., Songsanand P., Corkin S., Growdon J.H. Olfactory capacities in Alzheimer's Disease. Neurology 39, Suppl 1: 138, 1989; Rezek D.L. Olfactory deficits as a neurologic sign on dementia of the Alzheimer type. Arch. Neurol. 44: 1030-1032, 1987.

6. Koss E., Weiffenbach J.M., Haxby J.V., Friedland R.P. Olfactory detection and identification performance are dissociated in early Alzheimer's Disease. Neurology 38: 1228-1232, 1988.

7. Talamo B.R., Rudel R.A., Kosik K.S., Lee V.Y.M., Neff S., Adelman L., Kauer J.S. Pathological changes in olfactory neurons inn patients with Alzheimer's Disease. Aging 7: 11-14, 1976.

8. Erisi M.M., Wilcock G.K. The olfactory bulbs in Alzheimer's Disease. J. Neurol. Neurosurg. Psychiatr. 47: 56-60, 1984.

9. Hyman B.T., Arriagada P.V., Van Hoesen G.W. Pathological changes in the olfactory system in aging and Alzheimer's disease. Int. Study Group for the Pharmacology of Memory Disorders, in press. (1991)

10. Macrides F., Davis B.L. The olfactory bulb. In: Emson P.C. (ed.) Chemical Neuroanatomy, Raven Press, New York, pp. 391-426, 1983.

11. Macrides F., Davis B.L. The olfactory bulb. In: Emson P.C. (ed.) Chemical Neuroanatomy, Raven Press, New York, pp. 391-426, 1983

12. St. George-Hyslop P.H., Haines, J.L., Farrer L.A., et al. Genetic linkage studies suggest that Alzheimer's disease is not a single homogeneous disorder. Nature 347: 194-197, 1990.

13. Joachim C.L., Morris J.H., Selkoe D.J. Clinically diagnosed ALzheimer's disease: autopsy results in 150 cases. Ann. Neurol. 24: 50-56, 1988.

## Claims

1. A method for diagnosing disorders in which biological samples from normal and afflicted (abnormal) individuals are analyzed to generate patterns representative of molecular constituents of said samples, and characterized in that said method comprises creating a data base of frequency distribution patterns of constituents of samples from organisms having known categories of disorders and controls, and comparing the sample analysis for conformity to said frequency distribution patterns.

2. A method according to claim 1, wherein said samples comprise body fluid.

3. A method according to claim 2, wherein said body fluid is selected from cerebrospinal fluid, plasma, platelets, nasal mucosa, serum, saliva, and urine.

4. A method according to claim 1, wherein said disorder is selected from Alzheimer's Disease, Parkinson's Disease, Huntington's Disease, schizophrenia, Progressive Supernuclear Palsy, amyotrophic lateral sclerosis, senile dementis, tumors, carcinomas, and cardiovascular abnormalities.

5. A method according to claim 1, characterized by examining said patterns for chaotic or non-linear values.

6. A method according to claim 1, wherein said parameters are representative of at least one of the following selected from the group consisting of neurotransmitters, cofactors, precursors, metabolites, and associated compounds.

7. A method according to claim 1, wherein said disorder comprises Alzheimer's Disease, and the parameters measured comprise tyrosine and tryptophan peptide degradation fragments from beta amyloid.

8. A method according to claim 1, wherein said disorder comprises Parkinson's Disease, and the parameter measured comprises the analyte designated PO5.

## Patentansprüche

1. Verfahren zum Diagnostizieren von Erkrankungen, bei dem biologische Proben von normalen und an Beschwerden leidenden (anomalen) Einzelpersonen zur Erzeugung von molekularen Bestandteilen dieser Proben entsprechenden Mustern analysiert werden, dadurch gekennzeichnet, daß das Verfahren die Errichtung einer Datenbank aus Häufigkeitsverteilungsmustern der Bestandteile von Proben aus Organismen mit bekannten Krankheitskategorien und aus Kontrollpersonen umfaßt, sowie das Vergleichen der Probenanalyse auf Übereinstimmung mit den Häufigkeitsverteilungsmustern.

2. Verfahren nach Anspruch 1, dadurch gekennzeichnet, daß die Proben Körperflüssigkeit umfassen.

3. Verfahren nach Anspruch 2, dadurch gekennzeichnet, daß die Körperflüssigkeit aus Gehirn- und Rückenmarksflüssigkeit, Plasma, Blutplättchen, Nasenschleim, Serum, Speichel und Urin ausgewählt wird.

4. Verfahren nach Anspruch 1, dadurch gekennzeichnet, daß die Erkrankung aus der Alzheimer-Krankheit, der Parkinsonschen Krankheit, der Huntington-Krankheit, der Schizophrenie, der progressiven supernuklearen Paralyse, der amyotrophischen Lateralsklerose, der senilen Demenz, Tumoren, Karzinomen und kardiosvaskulären Anomalien ausgewählt wird.

5. Verfahren nach Anspruch 1, dadurch gekennzeichnet, daß die Muster auf ungeordnete oder nichtlineare Werte untersucht werden.

6. Verfahren nach Anspruch 1, dadurch gekennzeichnet, daß die Parameter wenigstens einem der folgenden Materialien entsprechen, die aus der aus Neurotransmittern, Cofaktoren, Vorläufern, Stoffwechselprodukten und damit verwandten Verbindungen bestehenden Gruppe ausgewählt werden.

7. Verfahren nach Anspruch 1, dadurch gekennzeichnet, daß die Erkrankung die Alzheimer Krankheit umfaßt, und daß die gemessenen Parameter Tyrosin- und Tryptophan-Peptidabbaufragmente von Beta-Amyloid umfassen.

8. Verfahren nach Anspruch 1, dadurch gekennzeichnet, daß die Erkrankung die Parkinsonsche Krankheit umfaßt, und daß der gemessene Parameter den mit PO5 bezeichneten Analyten umfaßt.


**Revendications**

1. Procédé de diagnostic de troubles, selon lequel on analyse des échantillons biologiques d'individus normaux et atteints (anormaux), pour générer des diagrammes représentatifs des constituants moléculaires desdits échantillons, et caractérisé en ce que ledit procédé comprend une création d'une base de données de diagrammes de distribution de fréquence de constituants des échantillons issus d'organismes ayant des catégories connues de troubles, et de témoins, et la comparaison de l'analyse d'échantillon eu égard à la conformité de ces diagrammes de distribution de fréquence.

2. Procédé selon la revendication 1, dans lequel lesdits échantillons comprennent un fluide corporel.

3. Procédé selon la revendication 2, dans lequel ledit fluide corporel est choisi parmi le fluide cérébro-spinal, le plasma, les plaquettes, la muqueuse nasale, le sérum, la salive et l'urine.

4. Procédé selon la revendication 1, dans lequel le trouble est choisi parmi la maladie d'Alzheimer, la maladie de Parkinson, la maladie d'Huntington , la schizophrénie, la paralysie supernucléaire évolutive, la sclérose latérale amyotrophique, la démence sénile, les tumeurs, les carcinomes et les anomalies cardiovasculaires.

5. Procédé selon la revendication 1, caractérisé en ce qu'on examine lesdits diagrammes pour des valeurs chaotiques ou non linéaires.

6. Procédé selon la revendication 1, dans lequel lesdits paramètres sont représentatifs d'au moins l'un des suivants choisis parmi les neurotransmetteurs, les cofacteurs, les précurseurs, les métabolites et les composés associés.

7. Procédé selon la revendication 1, dans lequel le trouble comprend la maladie d'Alzheimer, et les paramètres mesurés comprennent les fragments de dégradation peptidique tyrosine et tryptophane d'une amyloïde béta.

8. Procédé selon la revendication 1, dans lequel le trouble comprend la maladie de Parkinson, et le paramètre mesuré comprend la substance à analyser dénommée PO5.

HVA

FIG.1a

XAN/HVA

FIG.1b

TYR/TYR METABOLITES

FIG.1c

MHPG

FIG.1d

EP 0 567 564 B1

FIG.2

EP 0 567 564 B1

PROBABILITY OF AD AND NOT CONTROL

AD

CONTROL

NUMBER OF CASES

PROBABILITY

FIG.3

PROBABILITY OF AD AND NOT CONTROL

FIG.4

EP 0 567 564 B1

CHANNEL : 13 FULL SCREEN CURRENT 500nA

FIG.5

EP 0 567 564 B1

FIG.6A

## FIG.6B

FIG.7